# EUROPEAN PATENT SPECIFICATION

(11) **EP 1 800 632 B1**
(45) Date of publication and mention of the grant of the patent: **20.08.2008**
(21) Application number: 05425896.7
(22) Date of filing: 19.12.2005
(51) Int. Cl.: A61F 5/44, A61M 39/22, B65D 47/28

(54) **Body fluid collection bag and valve**
Beutel zur Aufnahme von Körperflüssigkeiten und Ventil
Poche de recueil de fluides corporels et soupape

(43) Date of publication of application: 27.06.2007
(73) Proprietor: SECURMED SPA, 36016 Thiene VI (IT)
(72) Inventor: Moretti, Gilberto, Massa Fiscaglia 44025 Ferrara (IT); Romagnoli, Paolo, 44100 Ferrara (IT)
(74) Representative: Bonini, Ercole

(56) References cited:
- EP-A1- 0 389 402
- WO-A-20/04103229
- FR-A1- 2 199 969
- GB-A- 2 390 547
- US-A- 4 055 179
- US-A- 5 228 646

## Description

The present invention concerns a medical device comprising a bag for collecting body fluids, in particular urine, and a valve suitable for attachment to said bag.

It is known that body fluids, like for example urine or blood, drained living creature or taken from the human body but also from the body of any other, in many cases are directly conveyed into a bag with suitable capacity, generally 2 litres.

Urine is drained via catheterism following various types of surgery carried out on bedridden patients.

The bag comprises a sealed container made of a plastic material in the form of a film, usually in polyvinylchloride (PVC), a delivery tube, one end of which is connected to a catheter of external or urethral type, applied to the patient's body, more precisely to the point from which the body fluid must be taken.

The opposite end of the delivery tube communicates with the inside of the container, to which it is permanently joined via connection means constituted, for example, by a radio frequency weld or pinch carried out on the PVC film so that it envelops a section of the delivery tube.

The bag also comprises a discharge tube of the body fluid stored in the container, said discharge tube being connected to the container in the same way as the delivery tube, and therefore communicating with the internal volume of the container itself.

The bag also comprises a valve or stopcock associated with the discharge tube and used by the health worker to discharge the fluid outside the container, into an environment or structure suited for this purpose, in a comfortable, practical, hygienic and effective way.

Moreover, the valve makes it possible to carry out a controlled discharge of the fluid, avoiding sudden and abrupt outflows from the bag, which inevitably involve for the health worker the risk of undesired, unhygienic and sometimes extremely dangerous contacts with the fluid.

The drainage of the fluid from the bag must obviously take place when the sealed container is completely full or, in any case, at the end of a working day, independently of the level reached by the fluid.

For hygienic reasons, the manufacturers of the medical devices in question recommend to consider the bag as disposable: once the fluid has been discharged from the sealed container, the bag must be definitively thrown away.

A first type of collection bags of the type briefly described above and presently available on the market is disclosed in the Patent applications FR 2 199 969 A1 and US 4,055,179 A, and comprises a valve, known as "push-pull", constituted by two coaxial tubular bodies, a first one of which is fixed to the container with a first end.

The second tubular body is externally and slidingly coupled to the second end of the first tubular body, so that two stable positions of the valve, corresponding to two operating configurations, are defined.

These are the closed position, in which the second tubular body is coupled to the first one in such a way as to close its outlet, and the open position, in which the second tubular body, following the exertion of a traction force, protrudes from the first of a length that is greater than in the closed position, thus opening the outlet of the first tubular body and allowing the body fluid to flow out.

The reciprocal position of the tubular bodies in the two operating configurations mentioned above is defined by stop means, for example annular projections, obtained on both tubular bodies.

According to another embodiment of the collection bags carried out according to the known art, which is disclosed in the Patent application WO 2004/103229 A, the valve is T-shaped and comprises a first tubular body that defines a first longitudinal axis, one end of which is joined to the sealed container, while the opposite end is in communication with the external environment.

The valve also comprises a second tubular body that develops along a longitudinal axis orthogonal to the longitudinal axis of the first tubular body, slidingly arranged inside a cylindrical element carried out as a single body integral to the first tubular body.

The second tubular body is provided, near the intermediate area of its side surface, with two coaxial through holes that, with the valve in closed position, face the inner wall of the cylindrical element.

A thrusting force is exerted by the user on the second tubular body, so that the valve is brought to the open position in which the two through holes are positioned coaxial to the first tubular body, thus allowing the fluid contained in the sealed container to be discharged.

Also in this case, the reciprocal positions of the tubular bodies in the two operating conditions described above are defined by stop means properly obtained on their internal and external surfaces.

The Patent application US 5, 228, 646 A discloses a different embodiment of the above mentioned valve, in which the closing and opening of the second tubular body are determined by the action of, respectively, a spring and retaining teeth.

Nevertheless, the body fluid collection bags carried out according to the known art are not without drawbacks.

The main drawback of the collection bags of the known type is represented by the fact that the user, after using them for the first time, can use them again once the stored fluid has been drained.

It is the construction form of the valves, originally designed exclusively to facilitate the disposal of the body fluid, that facilitates this operating method.

All of the known construction variants described above and, more generally, any collection bag carried out according to the known art and provided with mechanisms for regulating and controlling the discharge of the body fluid are characterised in that they allow the valve to be closed again even after it has been opened.

It often occurs that, following a wrong procedure, the user, starting from the open condition, closes the valve by applying a simple compression force to the second tubular body.

In this way the health worker, once having emptied the collection bag, leaves it applied to the patient via the catheter, even for more than 24 hours.

This happens notwithstanding the recommendations of the manufacturers that, in compliance with the rules aimed at guaranteeing the user's safety and good hygienic conditions, suggest to use the collection bags only once and to throw them away when they are completely full or, in any case, at the end of a working day.

Considering the type of product in question, which from a technical point of view is rather simple, the operating method described above exposes the patient, the health worker and any other person to serious risks, since the bag is a source of bacteria, viruses and other infections.

Collection bags designed so that a part of the valve will break have been launched on the market, in order to prevent them from being used twice.

In any case, also this solution has serious drawbacks, due to the uncontrolled outflow of body fluid during the discharge phase, which involves conditions that are highly critical and risky for the health worker and, in general, for the whole environment where the operation is carried out.

The present invention intends to overcome the drawbacks posed by the prior art which have just been mentioned.

In particular, the main aim of the present invention is to carry out a collection bag equipped with means suited to regulate the discharge of the body fluid that prevent the user from using the bag again after discharging the body fluid previously stored therein.

At the same time, the invention aims to allow the health worker to discharge the body fluid from the bag in absolutely safe and good hygienic conditions, in any case exceeding those allowed by equivalent bags of the known type.

The above mentioned goals have been achieved through the implementation of a body fluid collection bag comprising:
- a sealed container defining an internal volume that cannot be reached by a user;
- a delivery tube in communication with said internal volume of said container, suited to convey said fluid inside said container;
- a discharge tube in communication with said internal volume of said container, suited to discharge said fluid outside said container;
- a valve suited to regulate the discharge flow of said fluid, comprising:
   - a first tubular element associated with said discharge tube;
   - a second tubular element slidingly coupled to said first tubular element;
   - stop means, interposed between said tubular elements, suited to define for said valve at least one closed and one open position,
characterized in that said valve comprises definitive locking means, cooperating with said tubular elements, suited to prevent the reciprocal movement of said tubular elements when said valve is in said open position.

The subject of the present invention is also a valve for body fluid collection bags, suited to be associated with the discharge tube of said bag to regulate the discharge flow of said fluid and having the construction characteristics described above.

Advantageously, the invention carries out a body fluid collection bag that can be used only once, thus keeping to the recommendations of the manufacturers and at the same time respecting the minimum safety and hygiene conditions required for this type of medical devices.

Still to advantage, the discharge of the body fluid stored in the sealed container when this is completely full, or in any case at the end of a working day, is carried out by the health worker with no risk of contact with the fluid, in safer conditions than allowed by the known art.

Differently from the bags of the known type, the bag subject of the invention, in fact, does not involve the breakage of any of its components, especially in correspondence with the valve, during the body fluid discharge phase.

Therefore, the invention combines the positive effects determined on one hand by the impossibility to reuse the bag by closing the valve after discharging the fluid, and on the other hand by the way the fluid flows out of the sealed container towards the outside.

Once opened, the valve of the bag subject of the invention remains definitively in this position and at the same time the outflow of the body fluid is completely controlled.

Still to advantage, the valve used in the bag subject of the invention is simple to construct.

The aims and advantages described above, as well as others illustrated below, will be highlighted in greater detail in the description of a preferred embodiment of the invention, given indicatively with reference to the enclosed drawings, wherein:
- Figure 1 is an axonometric view of the bag subject of the invention in a first operating condition;
- Figure 2 is an axonometric view of the bag of Figure 1 in a second operating condition;
- Figure 3 is an exploded axonometric view, truncated and partially sectioned, of an enlarged detail of Figure 1;
- Figure 4 is a plan view, seen from below, of a component of the enlarged detail of Figure 3;
- Figures 5 and 6 show a longitudinal section of the detail of Figure 4 in two different operating conditions.

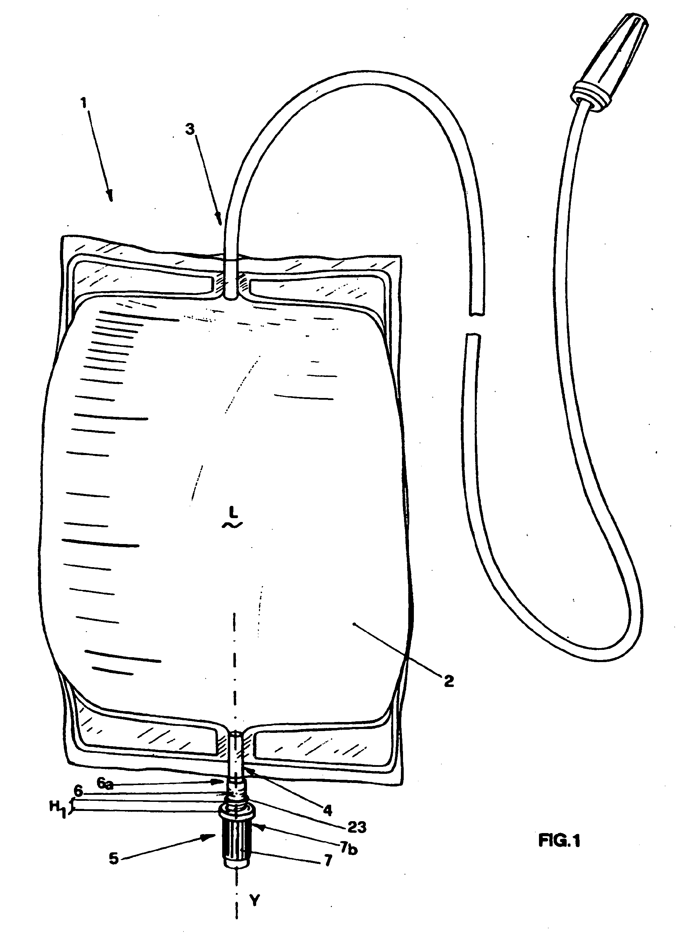

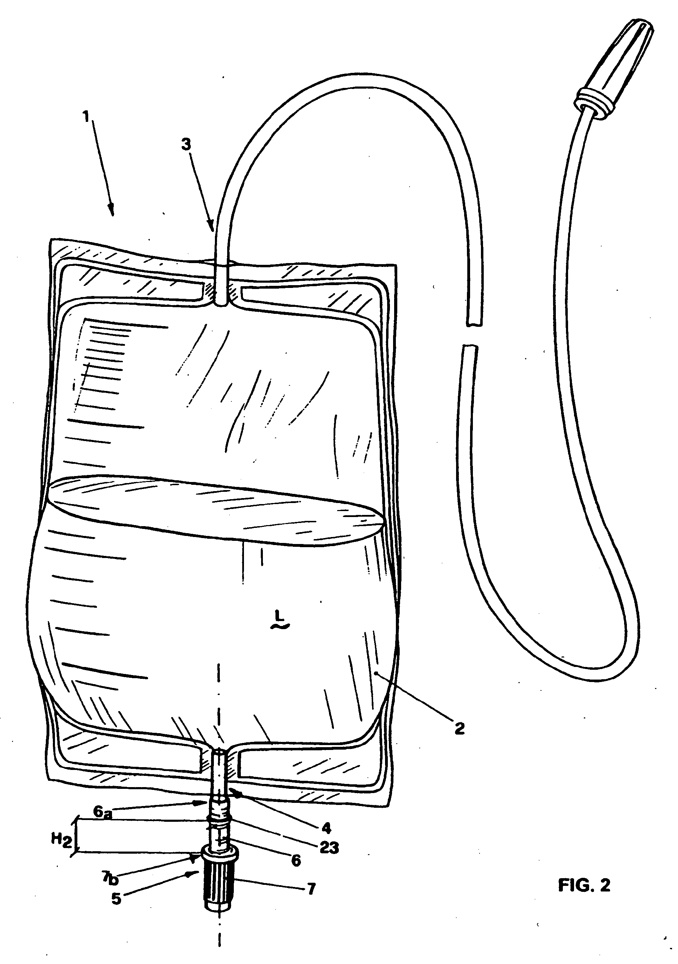

The bag subject of the invention is shown in Figures 1 and 2, where it is indicated as a whole by **1,** in two distinct operating conditions.

It is particularly suited to be used in hospitals, clinics and similar structures for collecting a body fluid L, for example urine.

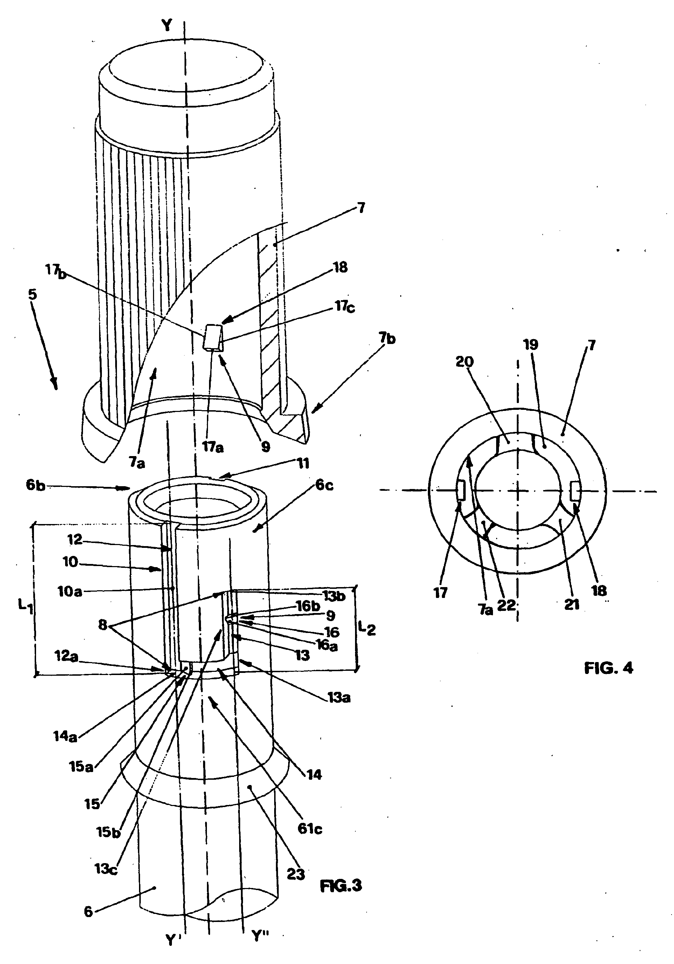

As it can be observed, the collection bag **1** comprises:
- a sealed container **2,** made of PVC, defining an internal volume that cannot be reached by a user;
- a delivery tube **3** in communication with the internal volume of the container **2,** suited to convey the fluid **L** inside said container;
- a discharge tube **4** in communication with the internal volume of the container **2,** suited to discharge the fluid L outside said container;
- a valve **5** suited to regulate the discharge flow of the fluid L, which in turn comprises:
   - a first tubular element **6,** preferably but not necessarily made of PVC, associated with the discharge tube **4,**
   - a second tubular element **7,** preferably made of polyethylene, slidingly coupled to said first tubular element **6;**
   - stop means, indicated as a whole by **8** and visible only in Figure 3, interposed between the tubular elements **6** and **7,** suited to define for the valve **5** one closed and one open position.

According to the invention, the valve **5** comprises definitive locking means, indicated as a whole by **9** and visible starting from Figure 3, cooperating with the tubular elements **6** and **7** and suited to prevent the reciprocal movement of the tubular elements **6** and **7** when the valve **5** is in the open position.

The delivery tube **3** and the discharge tube **4** are made of a plastic material, preferably but not necessarily **PVC,** and are joined to the container **2** via connection means, not visible but of the type known per se in these medical devices, for example a radio frequency pinched edge made on two opposite sides of the tubes **3** and **4.**

Figures 1 and 2 show that the first tubular element **6** is coupled to the discharge tube **4** at the level of the upper edge **6a** of the first tubular element **6.**

The tubular elements **6** and **7,** each having in a cross section a circular profile, are coaxial to each other and to the longitudinal axis **Y** defined by the discharge tube **4.**

As shown in Figure 3, the first tubular element **6** is provided with two articulated diametrically opposed grooves **10, 11** made on the outer wall **6c** starting from the lower edge **6b** of the first tubular element **6.**

From this point onwards and where not specified otherwise, for the sake of simplicity reference will be made to the articulated groove **10** only, and the statements made will be considered valid also for the articulated groove **11,** visible only partially in the figures.

The articulated groove **10** comprises:
- a first linear section **12** ending at the level of the lower edge **6b** of the first tubular element **6;**
- a second linear section **13** that develops parallel to the first linear section **12** and for a length **L₂** smaller than the length **L₁** of the first linear section **12**;
- a transverse rectilinear section 14 that connects two ends **12a**, **13a** of the linear sections **12, 13,** substantially obtained in the intermediate area **61c** of the outer wall **6c** of the first tubular element **6.**

Viewed from the front, the articulated groove **10** has a substantially U-shaped profile and, in any cross section, a polygonal profile.

The linear sections **12, 13** of the articulated groove **10** develop according to corresponding longitudinal directions **Y', Y"** substantially parallel to the longitudinal axis **Y** of the discharge tube **4.**

As to the stop means **8** that define the closed position of the valve **5,** they are constituted by the perimeter wall **14a** of the transverse rectilinear section **14** of the articulated groove **10.**

The stop means **8** that define, on the other hand, the open position of the valve **5,** are constituted by the upper wall **13b** of the second linear section **13** of the articulated groove **10.**

According to the preferred embodiment of the invention described herein, the locking means **9** comprise:
- two shaped wedges **15, 16** protruding from the bottom **10a** of the articulated groove **10**;
- two shaped teeth **17, 18,** both visible in greater detail in Figure 4, protruding from the inner wall **7a** of the second tubular element **7,** slidingly arranged inside the articulated grooves **10, 11,** respectively, and opposing the shaped wedges **15, 16** when a force is exerted on the second tubular element **7** in the direction opposite the direction according to which the shaped teeth **17, 18** previously overcame the shaped wedges **15, 16,** according to the explanation provided below.

Practically, the locking means **9** comprise a first shaped wedge **15,** arranged inside the rectilinear section **14** near the end **12a** of the first linear section **12** of the groove **10,** and a second shaped wedge **16,** positioned in a substantially intermediate area **13c** of the second linear section **13** of the groove **10.**

Each one of the shaped wedges **15, 16** presents, according to a cross section, a substantially triangular profile, with the base associated with the bottom **10a** of the groove **10** and the oblique sides diverging towards the outside starting from the bottom **10a** itself.

Each one of said shaped wedges **15, 16** has:
- a main plane surface **15a, 16a** that departs from the bottom **10a** according to a direction diverging towards the outside and incident to the longitudinal axis **Y** and defines a sort of ramp allowing the shaped tooth **17** to slide in the groove **10;**
- a secondary plane surface **15b, 16b** that departs from the bottom **10a** according to a direction substantially orthogonal to the bottom **10a** itself and defines a barrier that prevents the shaped tooth 17 from sliding in the groove **10;**
- two lateral plane surfaces opposite each other and in contact with the perimeter walls, for example that indicated by **14a**, of the groove **10.**

The shaped tooth **17** that fits into the articulated groove **10** and the shaped tooth **18** that fits into the articulated groove **11** are positioned near the end **7b** of the second tubular element **7** that is coupled to the first tubular element 6.

The shaped tooth **17,** as well as the shaped tooth indicated by **18,** presents, according to a diameter section, a substantially triangular profile, with the base associated with the inner wall **7a** of the second tubular element **7** and the oblique sides diverging towards the outside according to directions incident to the longitudinal axis **Y.**

The shaped tooth **17** is provided, at the level of the shorter oblique side, with a plane wall **17a** positioned adjacent to the secondary plane surface **16b** of the second wedge **16** to prevent the reciprocal translation of the tubular elements **6** and **7** along the longitudinal axis **Y** when a compression force is exerted on the second tubular element **7**, starting from the open position of the valve **5**.

Practically, the plane wall **17a** of the shaped tooth **17** resting against the secondary plane surface **16b** of the second wedge **16** stops any attempt to close the valve **5** after it has been opened to discharge the fluid **L** from the sealed container **2,** in order to prevent the collection bag **1** from being used again.

Obviously, in the articulated groove **11** the shaped tooth **18** rests against a corresponding second wedge, not shown in the attached drawings.

The shaped tooth **17** is also provided with two lateral plane walls **17b, 17c** opposite each other, one of which is positioned adjacent to the secondary plane surface **15b** of the first wedge **15** when the second tubular element **7** is set rotating clockwise around the longitudinal axis **Y** starting from the closed position of the valve **5.**

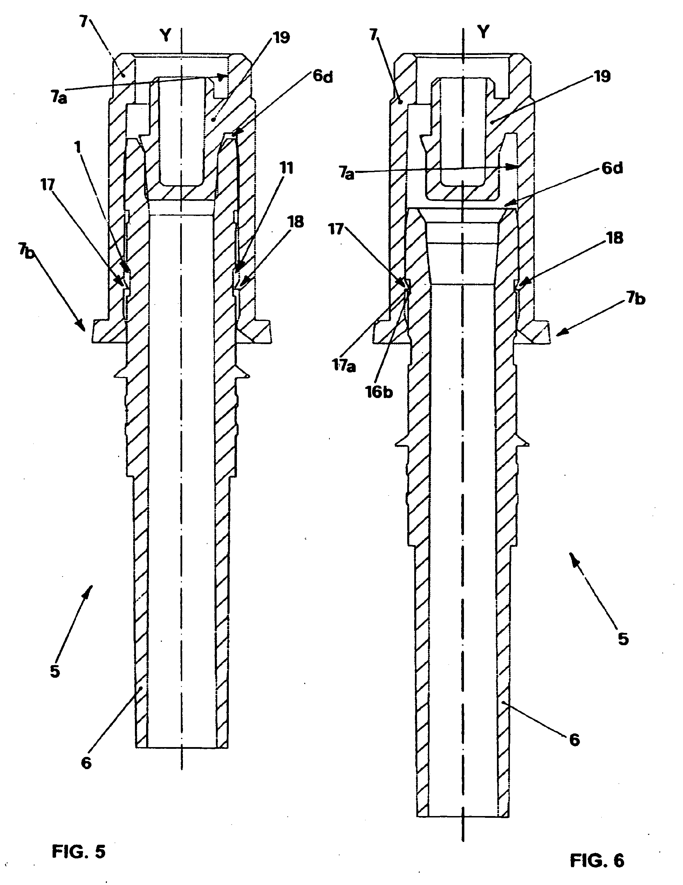

Figures 5 and 6 show that the second tubular element **7** is provided with a plug **19,** protruding from the inner wall **7a** of the second tubular element **7,** inserted in the outlet **6d** of the first tubular element **6** when the valve **5** is in the closed position.

In this case, the plug **19** is preferably connected to the second tubular element **7** via three radial ribs **20, 21, 22.**

It is important to point out that in further embodiments of the invention, not shown herein, the first tubular element may be provided with a single articulated groove on its outer wall.

Consequently, in this case the second tubular element will be provided on its inner wall with a single shaped tooth belonging to the locking means.

In the assembly phase, the user couples the second tubular element **7** to the first tubular element **6** introducing the shaped teeth **17, 18** inside the corresponding articulated groove **10, 11.**

More specifically, the shaped tooth **17** is inserted in the first linear section **12** until it meets the perimeter wall **14a** of the transverse rectilinear section **14** of the articulated groove **10.**

At this point, the user makes the second tubular element **7** rotate anticlockwise, so that during its movement inside the articulated groove **10** the shaped tooth **17** passes beyond the first wedge **15** and reaches the end **13a** of the second linear section **13,** remaining in any case within the transverse rectilinear section **14** of the groove **10.**

The same happens on the diametrically opposed side of the outer wall **6a** of the first tubular element **6** for the shaped tooth **18** inside the groove **11.**

The valve **5** obtained in this way is thus in the closed position shown in Figure 5, that is, the position in which the collection bag **1** with which it is connected is delivered to the health worker for use. Observe the distance **H₁** of the end **7b** of the second tubular element **7** from the ring **23** protruding from the outer wall **6c** of the first tubular element **6.**

During the day the collection bag **1** is completely filled with fluid **L,** as shown in Figure 1, which must be discharged outside the bag.

The health worker exerts a traction force on the second tubular element **7** and makes it slide along the longitudinal axis **Y,** thus increasing the length of the valve **5** in its whole and removing the plug **19** from the outlet 6d of the first tubular element **6.**

In this phase, the shaped tooth **17** slides inside the second linear section **13**, passing beyond the second wedge **16** until reaching the upper wall **13b**.

In this way, the valve **5** reaches the operating condition shown in Figure 6 and the fluid **L** is free to flow out of the sealed container **2** through the discharge tube **4**, as shown in Figure **2**, where it is possible to observe the distance **H₂** of the end **7b** of the second tubular element **7** from the ring **23**.

The health worker's attempt to bring the valve 5 back to the closed position in order to reuse the collection bag **1** fails due to the fact that the sliding movement of the second tubular element **7** along the longitudinal axis **Y** in the direction opposite the direction that determines the opening of the valve 5 is prevented by the second wedge **16** as the plane wall **17a** rests against the secondary plane surface **16b.**

In this way the aim to make a collection bag definitively and effectively disposable is achieved, with no need for the health worker to run the risks connected with contact with the fluid while emptying the sealed container.

The invention also prevents the health worker from opening the valve **5** in a way different from the one proposed, that is, by first rotating the second tubular element **7** clockwise and then exerting a traction force on it, making the shaped teeth **17, 18** cover the opposite path with respect to the path described for the production phase during the coupling of the tubular elements **6** and **7**.

In fact, the presence of the first wedge **15** locks the clockwise rotation around the longitudinal axis **Y** due to the opposition between the lateral plane wall **17b** and the secondary plane surface **15b**.

According to the above, it is therefore clear that the body fluid collection bag and the valve applied to it, which are the subject of the present invention, achieve the aims and offer the advantages mentioned above.

Upon implementation, modifications may be made to the collection bag that is the subject of the invention, consisting, for example, in a different shape of the tubular elements belonging to the valve.

Furthermore, the locking means may be of a type different from the one described as preferable, which does not affect the advantage offered by the present patent.

It is also important to point out that the first tubular element may be connected to the discharge tube via connection means, as in the embodiment described above, or may be carried out as a single body integral with it.

In the cases where the technical characteristics illustrated in the claims are followed by references, these have been added only with the aim to facilitate the comprehension of the claims themselves and therefore said references do not have any limiting effect on the degree of protection to be granted to each element they identify only by way of example.

All the variants described and mentioned, but not represented in the attached drawings, must be considered protected by the present patent, as they fall within the innovative concept expressed in the following claims.

## Claims

1. Collection bag (1) for body fluids (L) comprising:
- a sealed container (2) defining an internal volume that cannot be reached by a user;
- a delivery tube (3) in communication with said internal volume of said container (2), suited to convey said fluid (L) inside said container (2);
- a discharge tube (4) in communication with said internal volume of said container (2), suited to discharge said fluid (L) outside said container;
- a valve (5) suited to regulate the discharge flow of said fluid (L), comprising:
• a first tubular element (6) associated with said discharge tube (4);
• a second tubular element (7) slidingly coupled to said first tubular element (6);
• stop means (8), interposed between said tubular elements (6, 7), suited to define for said, valve (5) at least one closed and one open position,
**characterized in that** said valve (5) comprises definitive locking means (9), cooperating with said tubular elements (6, 7), suited to prevent the reciprocal movement of said tubular elements (6, 7) when said valve (5) is in said open position.

2. Bag (1) according to claim 1), **characterized in that** said first tubular element (6) is coupled to said discharge tube (4) at the level of the upper edge (6a) of said first tubular element (6).

3. Bag (1) according to claim 1), **characterized in that** said tubular elements (6, 7) are arranged coaxial to each other and to the longitudinal axis (Y) defined by said discharge tube (4).

4. Bag (1) according to claim 3), **characterized in that** said first tubular element (6) is provided with at least one articulated groove (10, 11) made on its outer wall (6c) starting from the lower edge (6b) of said first tubular element (6).

5. Bag (1) according to claim 4), **characterized in that** said articulated groove (10, 11) comprises:
- a first linear section (12) ending at the level of said lower edge (6b) of said first tubular element (6);
- a second linear section (13) that develops parallel to said first linear section (12) and for a length (L2) smaller than the length (L1) of said first linear section (12);
- a transverse rectilinear section (14) that connects two ends (12a, 13a) of said linear sections (12, 13), substantially obtained in the intermediate area (61 c) of said outer wall (6c) of said first tubular element (6).

6. Bag (1) according to claim 4), **characterized in that** said articulated groove (10, 11) when viewed from the front has a substantially U-shaped profile.

7. Bag (1) according to claim 4), **characterized in that** said articulated groove (10, 11) has a polygonal profile in any cross section.

8. Bag (1) according to claim 5), **characterized in that** said linear sections (12, 13) of said articulated groove (10, 11) develop along longitudinal directions (Y', Y") substantially parallel to said longitudinal axis (Y) of said discharge tube (4).

9. Bag (1) according to claim 8), **characterized in that** said stop means (8) defining said closed position of said valve (5) are constituted by the perimeter wall (14a) of said transverse rectilinear section (14) of said articulated groove (10, 11).

10. Bag (1) according to claim 8), **characterized in that** said stop means (8) defining said open position of said valve (5) are constituted by the upper wall (13b) of said second linear section (13) of said articulated groove (10,11).

11. Bag (1) according to claim 4), **characterized in that** said locking means (9) comprise:
- at least one shaped wedge (15, 16) protruding from the bottom (10a) of said articulated groove (10, 11);
- at least one shaped tooth (17) protruding from the inner wall (7a) of said second tubular element (7) and slidingly arranged inside said articulated groove (10, 11), suited to rest against said shaped wedge (15, 16) when a force is exerted on said second tubular element (7) in the direction opposite the direction according to which said shaped tooth (17) passed beyond said shaped wedge (15, 16).

12. Bag (1) according to claim 11), **characterized in that** said locking means (9) comprise a first shaped wedge (15), arranged in said rectilinear section (14) near said end (12a) of said first linear section (12) of said groove (10, 11), and a second shaped wedge (16), positioned in a substantially intermediate area (13c) of said second linear section (13) of said groove (10, 11).

13. Bag (1) according to claim 12), **characterized in that** each one of said shaped wedges (15, 16) presents, according to a cross section, a substantially triangular profile, with the base associated with said bottom (10a) of said groove (10, 11) and the oblique sides diverging towards the outside starting from said bottom (10a).

14. Bag (1) according to claim 12), **characterized in that** each one of said shaped wedges (15, 16) comprises:
- a main plane surface (15a, 16a) that departs from said bottom (10a) according to a direction diverging towards the outside and incident to said longitudinal axis (Y) and defines a sort of ramp allowing said shaped tooth (17) to slide in said groove (10, 11);
- a secondary plane surface (15b, 16b) that departs from said bottom (10a) according to a direction substantially orthogonal to said bottom (10a) and defines a barrier suited to prevent said shaped tooth (17) from sliding in said groove (10, 11);
- two lateral plane surfaces opposite each other and in contact with the perimeter walls of said groove (10, 11).

15. Bag (1) according to claim 11), **characterized in that** said shaped tooth (17) is arranged near the end (7b) of said second tubular element (7) that is coupled to said first tubular element (6).

16. Bag (1) according to claim 15), **characterized in that** said shaped tooth (17) presents, according to a diameter section, a substantially triangular profile, with the base associated with said inner wall (7a) of said second tubular element (7) and the oblique sides diverging towards the outside according to directions incident to said longitudinal axis (Y).

17. Bag (1) according to claim 16), **characterized in that** said shaped tooth (17) is provided, at the level of the shorter of said oblique sides, a plane wall (17c) suited to be arranged adjacent to said secondary plane surface (16b) of said second wedge (16) to prevent the reciprocal translation of said tubular elements (6, 7) along said longitudinal axis (Y) when a compression force is exerted on said second tubular element (7) starting from said open position of said valve (5).

18. Bag (1) according to claim 16), **characterized in that** said shaped tooth (17) is provided with to opposite plane lateral walls (17b 17c), one of which is suited to be arranged adjacent to said secondary plane surface (15b) of said first wedge (15) when said second tubular element (7) is set rotating around said longitudinal axis (Y) starting from said open position of said valve (5).

19. Bag (1) according to claim 1), **characterized in that** said second tubular element (7) is provided with a plug (19) protruding from the inner wall (7a) of said second tubular element (7) and inserted in the outlet (6d) of said first tubular element (6) when said valve (5) is in said closed position.

20. Bag (1) according to claim 19), **characterized in that** said plug (19) is connected to said second tubular element (7) via at least one radial rib (20, 21, 22).

21. Valve (5) for bags (1) for collecting body fluids (L), suited to be associated with the discharge tube (4) of said bag (1) to regulate the discharge flow of said fluid (L), comprising:
- a first tubular element (6) associated with said discharge tube (4);
- a second tubular element (7) slidingly coupled to said first tubular element (6);
- stop means (8), interposed between said tubular elements (6, 7), suited to define for said valve (5) at least one closed and one open position,
**characterized in that** it comprises definitive locking means (9), cooperating with said tubular elements (6, 7), suited to prevent the reciprocal movement of said tubular elements (6, 7) when said valve (5) is in said open position.

## Patentansprüche

1. Sammelbeutel (1) für Körperflüssigkeiten (L), umfassend:
- einen abgedichteten Behälter (2), der ein Innenvolumen definiert, das für einen Benutzer nicht zugänglich ist;
- einen Zuleitungsschlauch (3), der mit dem Innenvolumen des Behälters (2) in Kommunikation steht und dazu geeignet ist, die Flüssigkeit (L) in den Behälter (2) hinein zu leiten;
- einen Ableitungsschlauch (4), der mit dem Innenvolumen des Behälters (2) in Kommunikation steht und dazu geeignet ist, die Flüssigkeit (L) aus dem Behälter hinaus zu leiten;
- ein Ventil (5), das dazu geeignet ist, die Ableitungsströmung der Flüssigkeit (L) zu regulieren, umfassend:
• ein erstes röhrenförmiges Element (6), das dem Ableitungsschlauch (4) zugeordnet ist;
• ein zweites röhrenförmiges Element (7), das gleitend mit dem ersten röhrenförmigen Element (6) gekoppelt ist;
• Anschlagmittel (8), die zwischen den röhrenförmigen Elementen (6, 7) angeordnet sind und dazu geeignet sind, mindestens eine geschlossene und eine offene Stellung für das Ventil (5) zu definieren,
**dadurch gekennzeichnet, dass** das Ventil (5) definitive Sperrmittel (9) aufweist, die mit den röhrenförmigen Elementen (6, 7) zusammenwirken und dazu geeignet sind, die gegenläufige Bewegung der röhrenförmigen Elemente (6, 7) zu verhindern, wenn das Ventil (5) in der offenen Stellung ist.

2. Beutel (1) nach Anspruch 1), **dadurch gekennzeichnet, dass** das erste röhrenförmige Element (6) auf der Höhe des oberen Rands (6a) des ersten röhrenförmigen Elements (6) mit dem Ableitungsschlauch (4) gekoppelt ist.

3. Beutel (1) nach Anspruch 1), **dadurch gekennzeichnet, dass** die röhrenförmigen Elemente (6, 7) koaxial zueinander und zur Längsachse (Y), die vom Ableitungsschlauch (4) definiert ist, angeordnet sind.

4. Beutel (1) nach Anspruch 3), **dadurch gekennzeichnet, dass** das erste röhrenförmige Element (6) mindestens eine Gelenkrille (10, 11) aufweist, die an seiner Außenwand (6c), beginnend am unteren Rand (6b) des ersten röhrenförmigen Elements (6), ausgebildet ist.

5. Beutel (1) nach Anspruch 4), **dadurch gekennzeichnet, dass** die Gelenkrille (10, 11) Folgendes umfasst:
- einen ersten linearen Abschnitt (12), der auf der Höhe des unteren Rands (6b) des ersten röhrenförmigen Elements (6) endet;
- einen zweiten linearen Abschnitt (13), der sich parallel zum ersten linearen Abschnitt (12) über eine Länge (L2) ausdehnt, die kürzer als die Länge (L1) des ersten linearen Abschnitts (12) ist;
- einen quer verlaufenden, geradlinigen Abschnitt (14), der zwei Enden (12a, 13a) der linearen Abschnitte (12, 13) verbindet und im Wesentlichen im Mittelbereich (61c) der Außenwand (6c) des ersten röhrenförmigen Elements (6) erhalten ist.

6. Beutel (1) nach Anspruch 4), **dadurch gekennzeichnet, dass** die Gelenkrille (10, 11) von vorne betrachtet ein im Wesentlichen U-förmiges Profil aufweist.

7. Beutel (1) nach Anspruch 4), **dadurch gekennzeichnet, dass** die Gelenkrille (10, 11) in jedem Querschnitt ein polygonales Profil aufweist.

8. Beutel (1) nach Anspruch 5), **dadurch gekennzeichnet, dass** sich die linearen Abschnitte (12, 13) der Gelenkrille (10, 11) entlang von Längsrichtungen (Y', Y") im Wesentlichen parallel zur Längsachse (Y) des Ableitungsschlauchs (4) ausdehnen.

9. Beutel (1) nach Anspruch 8), **dadurch gekennzeichnet, dass** die Anschlagmittel (8), die die geschlossene Stellung des Ventils (5) definieren, durch die Umfangswand (14a) des quer verlaufenden, geradlinigen Abschnitts (14) der Gelenkrille (10, 11) gebildet sind.

10. Beutel (1) nach Anspruch 8), **dadurch gekennzeichnet, dass** die Anschlagmittel (8), die die offene Stellung des Ventils (5) definieren, durch die obere Wand (13b) des zweiten linearen Abschnitts (13) der Gelenkrille (10, 11) gebildet sind.

11. Beutel (1) nach Anspruch 4), **dadurch gekennzeichnet, dass** die Sperrmittel (9) Folgendes umfassen:
- mindestens einen Formkeil (15, 16), der vom Boden (10a) der Gelenkrille (10, 11) vorsteht;
- mindestens einen Formzahn (17), der von einer Innenwand (7a) des zweiten röhrenförmigen Elements (7) vorsteht, gleitend im Inneren der Gelenkrille (10, 11) angeordnet ist und dazu geeignet ist, am Formkeil (15, 16) anzuliegen, wenn an das zweite röhrenförmige Element (7) eine Kraft in die Richtung angelegt wird, die entgegengesetzt zu jener Richtung verläuft, in die der Formzahn (17) über den Formkeil (15, 16) hinaus bewegt wurde.

12. Beutel (1) nach Anspruch 11), **dadurch gekennzeichnet, dass** die Sperrmittel (9) einen ersten Formkeil (15), der im geradlinigen Abschnitt (14) in der Nähe des Endes (12a) des ersten linearen Abschnitts (12) der Rille (10, 11) angeordnet ist, und einen zweiten Formkeil (16), der in einem im Wesentlichen mittleren Bereich (13c) des zweiten linearen Abschnitts (13) der Rille (10, 11) angeordnet ist, umfassen.

13. Beutel (1) nach Anspruch 12), **dadurch gekennzeichnet, dass** jeder der Formkeile (15, 16) einem Querschnitt nach ein im Wesentlichen dreieckiges Profil aufweist, wobei die Grundlinie dem Boden (10a) der Rille (10, 11) zugeordnet ist und die schrägen Seiten vom Boden (10a) aus nach außen verlaufen.

14. Beutel (1) nach Anspruch 12), **dadurch gekennzeichnet, dass** jeder der Formkeile (15, 16) Folgendes umfasst:
- eine Hauptebenen-Oberfläche (15a, 16a), die sich ausgehend vom Boden (10a) einer nach außen verlaufenden und die Längsachse (Y) schneidenden Richtung folgend erstreckt und eine Art Rampe definiert, die dem Formzahn (17) das Gleiten in der Rille (10, 11) ermöglicht;
- eine Nebenebenen-Oberfläche (15b, 16b), die sich ausgehend vom Boden (10a) einer im Wesentlichen orthogonal zum Boden (10a) stehenden Richtung folgend erstreckt und eine Sperre definiert, die dazu geeignet ist, das Gleiten des Formzahns (17) in der Rille (10, 11) zu verhindern;
- zwei Seitenebenen-Oberflächen, die einander gegenüber liegen und mit den Umfangswänden der Rille (10, 11) in Berührung sind.

15. Beutel (1) nach Anspruch 11), **dadurch gekennzeichnet, dass** der Formzahn (17) in der Nähe des Endes (7b) des zweiten röhrenförmigen Elements (7), das mit dem ersten röhrenförmigen Element (6) gekoppelt ist, angeordnet ist.

16. Beutel (1) nach Anspruch 15), **dadurch gekennzeichnet, dass** der Formzahn (17) einem Durchmesserschnitt nach ein im Wesentlichen dreieckiges Profil aufweist, wobei die Grundlinie der Innenwand (7a) des zweiten röhrenförmigen Elements (7) zugeordnet ist und die schrägen Seiten Richtungen, die die Längsachse (Y) schneiden, folgend nach außen verlaufen.

17. Beutel (1) nach Anspruch 16), **dadurch gekennzeichnet, dass** der Formzahn (17) auf der Höhe der kürzeren der schrägen Seiten mit einer flachen Wand (17c) versehen ist, die dazu geeignet ist, benachbart zur Nebenebenen-Oberfläche (16b) des zweiten Keils (16) angeordnet zu werden, um die gegenläufige Bewegung der röhrenförmigen Elemente (6, 7) entlang der Längsachse (Y) zu verhindern, wenn eine Kompressionskraft auf das zweite röhrenförmige Element (7), ausgehend von der offenen Stellung des Ventils (5), ausgeübt wird.

18. Beutel (1) nach Anspruch 16), **dadurch gekennzeichnet, dass** der Formzahn (17) mit zwei gegenüberliegenden, flachen Seitenwänden (17b, 17c) ausgestattet ist, von denen eine dazu geeignet ist, benachbart zur Nebenebenen-Oberfläche (15b) des ersten Keils (15) angeordnet zu werden, wenn das zweite röhrenförmige Element (7), ausgehend von der offenen Stellung des Ventils (5), zur Drehung um die Längsachse (Y) eingestellt ist.

19. Beutel (1) nach Anspruch 1), **dadurch gekennzeichnet, dass** das zweite röhrenförmige Element (7) mit einem Stöpsel (19) ausgestattet ist, der von der Innenwand (7a) des zweiten röhrenförmigen Elements (7) vorsteht und in den Auslass (6d) des ersten röhrenförmigen Elements (6) eingeführt ist, wenn das Ventil (5) in seiner geschlossenen Stellung ist.

20. Beutel (1) nach Anspruch 19), **dadurch gekennzeichnet, dass** der Stöpsel (19) über mindestens eine radiale Rippe (20, 21, 22) mit dem zweiten röhrenförmigen Element (7) verbunden ist.

21. Ventil (5) für Beutel (1) zum Sammeln von Körperflüssigkeiten (L), das dazu geeignet ist, dem Ableitungsschlauch (4) des Beutels (1) zugeordnet zu werden, um die Ableitungsströmung der Flüssigkeit (L) zu regulieren, umfassend:
- ein erstes röhrenförmiges Element (6), das dem Ableitungsschlauch (4) zugeordnet ist;
- ein zweites röhrenförmiges Element (7), das gleitend mit dem ersten röhrenförmigen Element (6) gekoppelt ist;
- Anschlagmittel (8), die zwischen den röhrenförmigen Elementen (6, 7) angeordnet sind und dazu geeignet sind, mindestens eine geschlossene und eine offene Stellung für das Ventil (5) zu definieren,
**dadurch gekennzeichnet, dass** das Ventil (5) definitive Sperrmittel (9) aufweist, die mit den röhrenförmigen Elementen (6, 7) zusammenwirken und dazu geeignet sind, die gegenläufige Bewegung der röhrenförmigen Elemente (6, 7) zu verhindern, wenn das Ventil (5) in der offenen Stellung ist.

## Revendications

1. Sac de collecte (1) pour liquides organiques (L) comprenant:
- un conteneur hermétique (2) définissant un volume interne qui ne peut pas être atteint par l'utilisateur;
- un tuyau d'adduction (3) en communication avec ledit volume interne dudit conteneur (2), apte à transporter ledit liquide (L) à l'intérieur dudit conteneur (2);
- un tuyau d'évacuation (4) en communication avec ledit volume interne dudit conteneur (2), apte à évacuer ledit liquide (L) à l'extérieur dudit conteneur;
- une soupape (5) apte à régler le débit d'évacuation dudit liquide (L), comprenant:
• un premier élément tubulaire (6) associé avec ledit tuyau d'évacuation (4);
• un deuxième élément tubulaire (7) accouplé de manière coulissante audit premier élément tubulaire (6);
• des moyens d'arrêt (8), interposés entre lesdits éléments tubulaires (6, 7) aptes à définir pour ladite soupape (5) au moins une position de fermeture et une position d'ouverture,
**caractérisé en ce que** ladite soupape (5) comprend des moyens de blocage définitif (9), coopérant avec lesdits éléments tubulaires (6, 7), aptes à empêcher le mouvement réciproque desdits éléments tubulaires (6, 7) quand ladite soupape (5) se trouve dans ladite position d'ouverture.

2. Sac (1) selon la revendication 1), **caractérisé en ce que** ledit premier élément tubulaire (6) est accouplé audit tuyau d'évacuation (4) à hauteur du bord supérieur (6a) dudit premier élément tubulaire (6).

3. Sac (1) selon la revendication 1), **caractérisé en ce que** lesdits éléments tubulaires (6, 7) sont positionnés coaxialement l'un par rapport à l'autre et à l'axe longitudinal (Y) défini par ledit tuyau d'évacuation (4).

4. Sac (1) selon la revendication 3), **caractérisé en ce que** ledit premier élément tubulaire (6) présente au moins une rainure articulée (10, 11) réalisée sur sa paroi extérieure (6c) à partir du bord inférieur (6b) dudit élément tubulaire (6).

5. Sac (1) selon la revendication 4), **caractérisé en ce que** ladite rainure articulée (10, 11) comprend:
- une première partie linéaire (12) se terminant à hauteur dudit bord inférieur (6b) dudit premier élément tubulaire (6);
- une deuxième partie linéaire (13) qui se développe parallèlement à ladite première partie linéaire (12) et pour une longueur (L2) inférieure à la longueur (L1) de ladite première partie linéaire (12);
- une partie rectiligne transversale (14) qui relie deux extrémités (12a, 13a) desdites parties linéaires (12, 13), essentillement obtenues dans la zone intermédiaire (61 c) de ladite paroi extérieure (6c) dudit premier élément tubulaire (6).

6. Sac (1) selon la revendication 4), **caractérisé en ce que** ladite rainure articulée (10, 11) présente en vue de face un profil essentiellement en U.

7. Sac (1) selon la revendication 4), **caractérisé en ce que** ladite rainure articulée (10, 11) présente un profil polygonal dans une section quelconque.

8. Sac (1) selon la revendication 5), **caractérisé en ce que** lesdites parties linéaires (12, 13) de ladite rainure articulée (10, 11) se développent lelong des directions longitudinales (Y', Y") esentiellement parallèles audit axe longitudinal (Y) dudit tuyau d'évacuation (4).

9. Sac (1) selon la revendication 8), **caractérisé en ce que** lesdits moyens d'arrêt (8) définissant ladite position de fermeture de ladite soupape (5) se composent de la paroi périmétrale (14a) de ladite partie rectiligne transversale (14) de ladite rainure articulée (10, 11).

10. Sac (1) selon la revendication 8), **caractérisé en ce que** ledits moyens d'arrêt (8) définissant ladite position d'ouverture de ladite soupape (5) se composent de la paroi supérieure (13b) de ladite deuxième partie linéaire (13) de ladite rainure articulée (10, 11).

11. Sac (1) selon la revendication 4), **caractérisé en ce que** lesdits moyens de blocage (9) comprennent:
- au moins un coin galbé (15, 16) saillant du fond (10a) de ladite rainure articulée (10, 11);
- au moins une dent galbée (17) saillant de ladite paroie interne (7a) dudit deuxième élément tubulaire (7) et positionnée de manière coulissante à l'intérieur de ladite rainure articulée (10, 11), apte à s'appuyer contre ledit coin galbé (15, 16) quand une force est exercée sur ledit deuxième élément tubulaire (7) dans la direction opposée à la direction selon laquelle ladite dent galbée (17) a dépassé ledit coin galbé (15, 16).

12. Sac (1) selon la revendication 11), **caractérisé en ce que** lesdits moyens de blocage (9) comprennent un premier coin galbé (15), positionné dans ladite partie rectiligne (14) près de ladite extrémité (12a) de ladite première partie linéaire (12) de la dite rainure (10, 11), et un deuxième coin galbé (16), positionné dans une zone essentiellement intermédiaire (13c) de ladite deuxième partie linéaire (13) de ladite rainure (10, 11).

13. Sac (1) selon la revendication 12), **caractérisé en ce que** chacun desdits coins galbés (15, 16) présente, selon une section, un profil essentiellement triangulaire, avec la base associée audit fond (10a) de ladite rainure (10, 11) et les côtés obliques divergeant vers l'extérieur à partir dudit fond (10a).

14. Sac (1) selon la revendication 12), **caractérisé en ce que** chacun desdits coins galbés (15, 16) comprend :
- une surface plane principale (15a, 16a) qui part dudit fond (10a) selon une direction divergeant vers l'extérieur et incidente sur ledit axe longitudinal (Y) et qui définit une sorte de rampe permettant à ladite dent galbée (17) de coulisser dans ladite rainure (10, 11);
- une surface plane secondaire (15b, 16b) qui part dudit fond (10a) selon une direction essentiellement orthogonale audit fond (10a) et qui définit une barrière apte à empêcher que ladite dent galbée (17) puisse coulisser dans ladite rainure (10, 11);
- deux surfaces planes latérales opposées l'une par rapport à l'autre et en contact avec les parois périmétrales de ladite rainure (10, 11).

15. Sac (1) selon la revendication 11), **caractérisé en ce que** ladite dent galbée (17) est positionnée près de l'extrémité (7b) dudit deuxième élément tubulaire (7) qui s'accouple audit premier élément tubulaire (6).

16. Sac (1) selon la revendication 15), **caractérisé en ce que** ladite dent galbée (17) présente, selon une section diamétrale, un profil essentiellement triangulaire, avec la base associée avec ladite paroi interne (7a) dudit deuxième élément tubulaire (7) et les côtés obliques divergeant vers l'extérieur selon des directions incidentes sur ledit axe longitudinal (Y).

17. Sac (1) selon la revendication 16), **caractérisé en ce que** ladite dent galbée (17) présente, à hauteur du côté le plus petit desdits côtés obliques, une paroi plane (17c) apte à être positionnée de manière adjacente à ladite surface plane secondaire (16b) dudit deuxième coin (16) afin d'empêcher la translation réciproque desdits éléments tubulaires (6, 7) le long dudit axe longitudinal (Y) quand une force de compression est exercée sur ledit deuxième élément tubulaire (7) à partir de ladite position d'ouverture de ladite soupape (5).

18. Sac (1) selon la revendication 16), **caractérisé en ce que** ladite dent galbée (17) présente deux parois planes latérales (17b, 17c), l'une de celles-ci étant indiquée pour être positionnée de manière adjacente à ladite surface plane secondaire (15b) dudit coin (15) quand ledit deuxième élément tubulaire (7) est positionné en rotation autour dudit axe longitudinal (Y) à partir de ladite position d'ouverture de ladite soupape (5).

19. Sac (1) selon la revendication 1), **caractérisé en ce que** ledit deuxième élément tubulaire (7) présente un bouchon (19) saillant de la paroi interne (7a) dudit deuxième élément tubulaire (7) et inséré dans l'orifice de sortie (6d) dudit premier élément tubulaire (6) quand ladite soupape (5) se trouve dans ladite position de fermeture.

20. Sac (1) selon la revendication 19), **caractérisé en ce que** ledit bouchon (19) est relié audit deuxième élément tubulaire (7) à travers au moins une nervure radiale (20, 21, 22).

21. Soupape (5) pour sacs (1) pour la collecte de liquides organiques (L), apte à être associée au tuyau d'évacuation (4) dudit sac (1) pour régler le débit d'évacuation dudit liquide (L), comprenant:
- un premier élément tubulaire (6) associé au tuyau d'évacuation (4);
- un deuxième élément tubulaire (7) relié de manière coulissante audit premier élément tubulaire (6);
- des moyens d'arrêt (8), interposés entre lesdits éléments tubulaires (6, 7), aptes à définir pour ladite soupape (5) au moins une position de fermeture et une position d'ouverture,
**caractérisée en ce qu'**elle comprend des moyens de blocage définitif (9), coopérant avec ledits éléments tubulaires (6, 7), aptes à empêcher le mouvement réciproque desdits éléments tubulaires (6, 7) quand ladite soupape (5) se trouve dans ladite position d'ouverture.
